# EUROPEAN PATENT SPECIFICATION

(11) **EP 2 753 933 B1**
(45) Date of publication and mention of the grant of the patent: **09.08.2017**
(21) Application number: 12766763.2
(22) Date of filing: 07.09.2012
(51) Int. Cl.: G01N 33/574, G01N 33/74

(54) **USE OF HUMAN PAPILLOMAVIRUS STATUS IN ESTABLISHING USE OF AN AGENT THAT BINDS EGFR IN THE TREATMENT OF CANCER**
VERWENDUNG DES HUMAN-PAPILLOMAVIRENSTATUS BEI DER BESTIMMUNG DER VERWENDUNG EINES MITTELS ZUR BINDUNG VON EGFR BEI DER BEHANDLUNG VON KREBS
UTILISATION DE L'ÉTAT D'UN PAPILLOMAVIRUS HUMAIN POUR ÉTABLIR L'UTILISATION D'UN AGENT QUI FIXE L'EGFR DANS LE TRAITEMENT DU CANCER

(30) Priority: 09.09.2011 US 201161533082 P
(43) Date of publication of application: 16.07.2014
(73) Proprietor: Amgen Inc., Thousand Oaks, California 91320-1799 (US)
(72) Inventor: WIEZOREK, Jeffrey, Scott, Malibu, CA 90265 (US); BACH, Bruce, Allen, Thousand Oaks, CA 91320 (US)
(74) Representative: Grünecker Patent- und Rechtsanwälte PartG mbB
(86) International application number: PCT/US2012/054137
(87) International publication number: WO 2013/066491

(56) References cited:
- EP-A1- 2 371 369
- SILKE TRIBIUS ET AL: "Impact of HPV status on treatment of squamous cell cancer of the oropharynx: What we know and what we need to know", CANCER LETTERS, NEW YORK, NY, US, vol. 304, no. 2, 2 February 2011 (2011-02-02), pages 71-79, XP028167652, ISSN: 0304-3835, DOI: 10.1016/J.CANLET.2011.02.002 [retrieved on 2011-02-05]
- LE Q T ET AL: "Integrating Biologically Targeted Therapy in Head and Neck Squamous Cell Carcinomas", SEMINARS IN RADIATION ONCOLOGY, SAUNDERS, PHILADELPHIA, PA, US, vol. 19, no. 1, 1 January 2009 (2009-01-01), pages 53-62, XP025684444, ISSN: 1053-4296, DOI: 10.1016/J.SEMRADONC.2008.09.010 [retrieved on 2008-11-24]
- CHAU NICOLE G ET AL: "The association between EGFR variant III, HPV, p16, c-MET, EGFR gene copy number and response to EGFR inhibitors in patients with recurrent or metastatic squamous cell carcinoma of the head and neck", HEAD & NECK ONCOLOGY, BIOMED CENTRAL LTD, LONDON, UK, vol. 3, no. 1, 27 February 2011 (2011-02-27), page 11, XP021091362, ISSN: 1758-3284, DOI: 10.1186/1758-3284-3-11

## Description

### FIELD OF THE INVENTION

The instant disclosure relates to methods of using Human Papillomavirus (HPV) negative status as a predictive indicator in patients having at least one type of cancer, including cancers characterized by the presence of a tumor, such as a head and neck squamous cell carcinoma (HNSCC) tumor, particularly in the context of a therapeutic regimen involving an anti-epidermal growth factor receptor (EGFr) specific binding agent. The instant disclosure also relates to methods of treating patients having a tumor, such as a HNSCC tumor, using an anti-EGFr specific binding agent, and to methods of stratifying such patients on the basis of HPV negative status. The anti-EGFR specific binding agent can be, *e.g*., an antibody, such as a monoclonal antibody.

### BACKGROUND OF THE INVENTION

Certain applications of monoclonal antibodies in cancer therapy rely on the ability of the antibody to specifically deliver to the cancerous tissues cytotoxic effector functions such as immune-enhancing isotypes, toxins or drugs. Another alternative approach is to utilize monoclonal antibodies to directly affect the survival of tumor cells by depriving them of essential extracellular proliferation signals, such as those mediated by growth factors through their cell receptors. One attractive target in this approach is the epidermal growth factor receptor (EGFr), which binds EGF and can also bind transforming growth factor α (TGFα) (see, *e.g*., Ullrich et al., Cell 61:203-212, 1990; Baselga et al., Pharmacol. Ther. 64: 127-154, 1994; Mendelsohn et al., in Biologic Therapy of Cancer 607-623, Philadelphia: J.B. Lippincott Co., 1995; and Fan et al., Curr. Opin. Oncol. 10: 67-73, 1998). Binding of EGF or TGFα to EGFr, a 170 kDa transmembrane cell surface glycoprotein, triggers a cascade of cellular biochemical events, including EGFr autophosphorylation and internalization, which culminates in cell proliferation (see, *e.g*., Ullrich et al., Cell 61:203-212, 1990).

Monoclonal antibodies specific to human EGFr, have been generated from mice and rats (see, *e.g*., Baselga et al., Pharmacol. Ther. 64: 127-154, 1994; Mendelsohn et al., in Biologic Therapy of Cancer pp. 607-623, Philadelphia: J.B. Lippincott Co., 1995; Fan et al., Curr. Opin. Oncol. 10: 67-73, 1998; Modjtahedi et al., Intl. J. Oncology 4: 277-296, 1994). Some of those antibodies, such as the mouse antibodies 108, 225 (see, e.g., Aboud-Pirak et al., J. Natl. Cancer Inst. 80: 1605-1611, 1988) and 528 (see, e.g., Baselga et al., Pharmacol. Ther. 64: 127-154, 1994; Mendelsohn et al., in Biologic Therapy of Cancer pp. 607-623, Philadelphia: J.B. Lippincott Co., 1995) or the rat ICR16, ICR62 and ICR64 (see, e.g., Modjtajedi et al., Intl. J. Oncology 4: 277-296, 1994; Modjtahedi et al., Br. J. Cancer 67:247-253, 1993; Modjtahedi et al., Br. J. Cancer 67: 254-261, 1993) monoclonal antibodies, were evaluated extensively for their ability to affect tumor growth in xenograft mouse models. A chimeric version of the 225 monoclonal antibody (C225), in which the mouse antibody variable regions are linked to human constant regions, exhibited an *improved in vivo* anti-tumor activity but only at high doses (see, e.g., Goldstein et al., Clinical Cancer Res. 1: 1311-1318, 1995; Prewett et al., J. Immunother. Emphasis Tumor Immunol. 19: 419-427, 1996). This antibody ultimately became cetuximab (Erbitux®, Eli Lilly).

Certain advances in the biological arts have made it possible to produce a fully human anti-EGFr antibodies. Using mice transgenic for human immunoglobulin genes (Xenomouse® technology, Abgenix, Inc.), human antibodies specific for human EGFr were developed (see, *e.g.,* Mendez, Nature Genetics, 15: 146-156, 1997; Jakobovits, Adv. Drug Deliv. Rev., 31(1-2): 33-42, 1998; Jakobovits, Expert Opin. Invest. Drugs, 7(4): 607-614, 1998; Yang et al., Crit. Rev. Oncol. Hematol. 38(1):17-23, 2001; WO98/24893; WO 98/50433). One such antibody, panitumumab (Vectibix®, Amgen Inc), a human IgG2 monoclonal antibody with an affinity of 5 x 10-11 M for human EGFr, has been shown to block binding of EGF to the EGFr, to block receptor signaling, and to inhibit tumor cell activation and proliferation *in vitro* (see, *e.g*., WO98/50433; See, *e.g*., U.S. Patent No. 6,235,883). Studies in athymic mice have demonstrated that panitumumab also has *in vivo* activity, not only preventing the formation of human epidermoid carcinoma A431 xenografts in athymic mice, but also eradicating already-established large A431 tumor xenografts (see, *e.g*., Yang et al., Crit. Rev. Oncol. Hematol. 38(1):17-23, 2001; Yang et al., Cancer Res. 59(6): 1236-43, 1999). Panitumumab has been considered for the treatment of renal carcinoma, colorectal adenocarcinoma, prostate cancer, and non small cell squamous lung carcinoma, among other cancers (see, *e.g*., U.S. Patent Publication No. 2004/0033543). Panitumumab has been approved by the US Food & Drug Administration to treat certain patients with metastatic colorectal cancer.

Papillomaviruses induce benign, dysplastic and malignant hyperproliferations of skin and mucosal epithelium (see, *e.g.,* Mansur and Androphy, (1993) Biochim Biophys Acta 1155:323-345; Pfister (1984) Rev. Physiol. Biochem. Pharmacol. 99:111-181; and Broker et al. (1986) Cancer Cells 4:17-36, for reviews of the molecular, cellular, and clinical aspects of the papillomaviruses). While most HPV-induced epithelial lesions are benign, lesions arising from certain papillomavirus types e.g., HPV-16 and HPV-18, can undergo malignant progression.

The role of HPV in the development and natural history of various cancers, including head and neck cancers, has been studied. See, *e.g*., Joseph & Pai, (2011) ASCO; Lassen et al., (2009) J Clin Onc. 27:1992-98; Perrone et al., (2006) Human Cancer Biol. 12:6643-6651; Stetlow et al., (2010) Am JSurg. Path. 34:e15-e24; Ihloff et al., (2010) Oral Onc. 46:705-11; Klussman et al, (2003) Am J Path. 162:747-53. More particularly, Lassen et al. demonstrate that p16^{INK4A} is correlated with HPV infection and thus exhibits a prognostic effect in HPV positive HNSCC patients. Perrone et al. demonstrated that there are various forms of HNSCC, including HPV-associated HNSCC and environmentally-driven HNSCC; each of these forms of HNSCC show marked differences in terms of p53 status and EGFr gene amplification. Stetlow et al. described the histopathological parameters in HPV positive HNSCC patients. Klussman et al. demonstrated the molecular and histopathological separation between HPV positive and HPV negative HNSCC patients. Finally, Ilhoff et al. reviewed recent clinical studies supporting the idea that HPV-positive tumor status can serve as a prognostic factor associated in HNSCC patients. Similarly, Tribius et al., Cancer Letters, vol. 204, no. 2, 2 February 2011, pages 71-79, discussed that patients with oropharyngeal cancer show better survival when having HPV positive tumors.

HPV positive locally advanced HNSCC patients have been observed to possess a different spectrum of DNA mutations, and a better prognosis than HPV negative HNSCC patients. The referenced studies may or may not collectively point to a prognostic role of HPV status in HNSCC patients but, regardless of whether HPV status is or is not a reliable *prognostic* indicator, until the present disclosure there has been no discussion of HPV status as a *predictive* indicator in HNSCC patients for a drug, *e.g*., panitumumab or cetuximab, nor has there been any demonstration of an enhanced effect of a drug, *e.g*., panitumumab or cetuximab, on the overall survival and/or progression-free survival of HPV negative HNSCC patients.

In some trials EGFr inhibitors have been shown to generate sufficient survival benefit even in unselected populations, but in others there was no substantial benefit. Even in the case of some approved EGFr inhibitors it has become more and more clear that efficient and reliable tests are of benefit in identifying those patients that might meaningfully benefit from treatment with EGFR inhibitors and those patients that are not likely to meaningfully benefit from such therapy. See, *e.g.*, Ladanyi et al., Mod Pathol. 2008 May; 21 Suppl 2:S16-22. As described herein, the HPV status of a tumor in HNSCC patients can serve as such an indicator. In accordance with the instant disclosure, it has now been found that EGFr inhibitors (such as panitumumab) improve progression-free survival and overall survival of patients having HPV negative tumors, particularly HNSCC tumors. Consistent with this data, the instant disclosure also relates to screeing a patient to determine whether administering an EGFr inhibitor to the patient will provide a therapeutic benefit.

### SUMMARY

The present invention relates to the following items:
1. A method of predicting whether a patient having a tumor will benefit from treatment comprising an agent that specifically binds to an EGFr polypeptide, comprising determining whether the patient's tumor is HPV positive or HPV negative, wherein if the patient's tumor is HPV negative, the patient is predicted to beneft from treatment with an agent that specifically binds to an EGFr polypeptide, wherein the agent that specifically binds to an EGFr polypeptide is an antibody to EGFr.
2. The method of item 1, wherein the determining comprises determining the presence or absence of p16^{INK4A} in a tumor sample obtained from the patient, wherein the presence of p16^{INK4A} indicates that the patient is HPV positive and the absence of p16^{INK4A} indicates that the patient is HPV negative.
3. The method of item 1, wherein the antibody to EGFr is panitumumab.
4. The method of item 2, wherein the determining the presence or absence of p16^{INK4A} in a tumor comprises performing an immunohistochemistry (IHC) assay to identify the presence or absence of p16^{INK4A} in a sample of the tumor.
5. The method of item 1, wherein the tumor is a locally advanced tumor or a recurrent metastatic tumor; preferably wherein the tumor is selected from the group consisting of an oropharangeal tumor, a larynx tumor, a tumor of the oral cavity and a tumor of the hypopharynx.
6. A monoclonal antibody that specifically binds to an EGFr polypeptide for use in a method of prolonging the progression-free and/or overall survival of a patient having a tumor that has been determined to be HPV negative, wherein the tumor is determined to be HPV negative by determining the presence or absence of p16^{INK4A} in a tumor obtained from the patient, wherein the absence of p16^{INK4A} indicates that the patient is HPV negative.
7. A monoclonal antibody that specifically binds to an EGFr polypeptide for use in the treatment of a patient having a tumor that has been determined to be HPV negative, wherein the tumor is determined to be HPV negative by determining the presence or absence of p16^{INK4A} in a tumor obtained from the patient, wherein the absence of p16^{INK4A} indicates that the patient is HPV negative.
8. The antibody that specifically binds to an EGFr polypeptide for the use according to item 6 or 7, wherein the antibody to EGFr is panitumumab.
9. The antibody that specifically binds to an EGFr polypeptide for the use according to item 6 or 7, wherein the determining the presence or absence of p16^{INK4A} in a tumor comprises performing an immunohistochemistry (IHC) assay to identify the presence or absence of p16^{INK4A} in a sample of the tumor.
10. The antibody that specifically binds to an EGFr polypeptide for the use according to item 6 or 7, wherein the tumor is a locally advanced tumor or a recurrent metastatic tumor; preferably wherein the tumor is selected from the group consisting of an oropharangeal tumor, a larynx tumor, a tumor of the oral cavity and a tumor of the hypopharynx.
11. The antibody that specifically binds to an EGFr polypeptide for the use according to item 6 or 7, wherein chemotherapy is used in addition to the antibody that specifically binds to an EGFr polypeptide.
12. A method of stratifying a population of patients having a tumor comprising:
   (a) determining whether the patient's tumor is HPV positive or HPV negative; and
   (b) selecting patients whose tumors are HPV negative for treatment with a therapy comprising an agent that specifically binds to an EGFr polypeptide.
13. The method of item 12, wherein the determining comprises determining the presence or absence of p16^{INK4A} in a tumor obtained from the patient, wherein the presence of p16^{INK4A} indicates that the patient is HPV positive and the absence of p16^{INK4A} indicates that the patient is HPV negative.
14. The method of item 12, wherein the agent that specifically binds to an EGFr polypeptide is an antibody to EGFr; preferably wherein the antibody to EGFr is panitumumab.
15. The method of item 12, wherein the determining the presence or absence of p16^{INK4A} in a tumor comprises performing an immunohistochemistry (IHC) assay to identify the presence or absence of p16^{INK4A} in a sample of the tumor.
16. The method of item 12, wherein the tumor is a locally advanced tumor or a recurrent metastatic tumor; preferably wherein the tumor is selected from the group consisting of an oropharangeal tumor, a larynx tumor, a tumor of the oral cavity and a tumor of the hypopharynx.

### BRIEF DESCRIPTION OF THE FIGURES

Figure 1 is a Kaplan-Meier survival curve showing the effect on overall survival of patients receiving panitumumab and chemotherapy versus chemotherapy alone in HPV negative subjects.
Figure 2 is a Kaplan-Meier survival curve showing the effect on overall survival of patients receiving panitumumab and chemotherapy versus chemotherapy alone in HPV positive subjects.
Figure 3 is a Kaplan-Meier survival curve showing the effect on progression-free survival of patients receiving panitumumab and chemotherapy versus chemotherapy alone in HPV negative subjects.
Figure 4 is a Kaplan-Meier survival curve showing the effect on progression-free survival of patients receiving panitumumab and chemotherapy versus chemotherapy alone in HPV positive subjects.

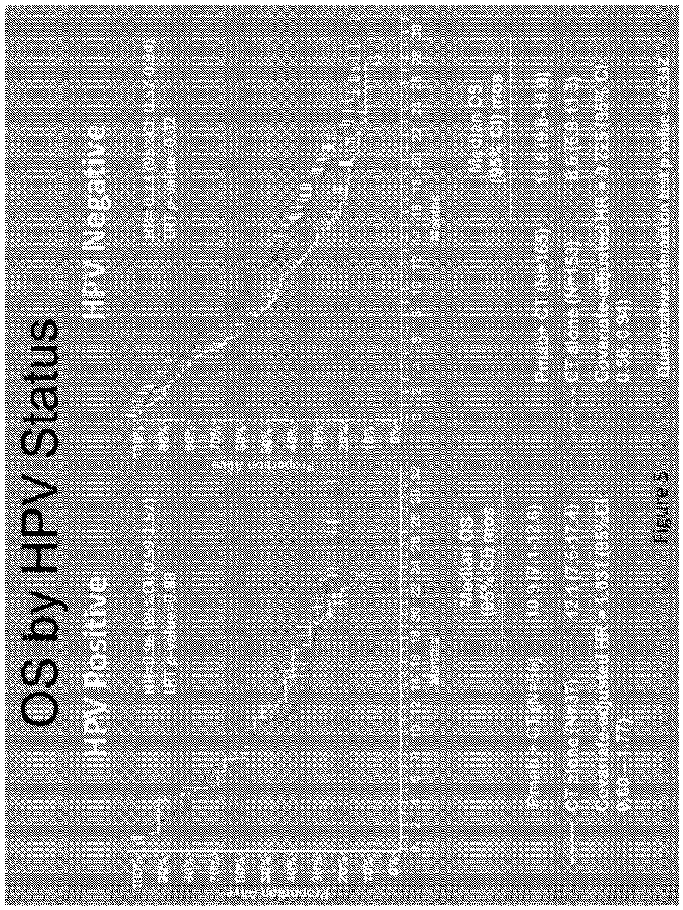

Figure 5 is a plot showing the effect of HPV status on overall survival.

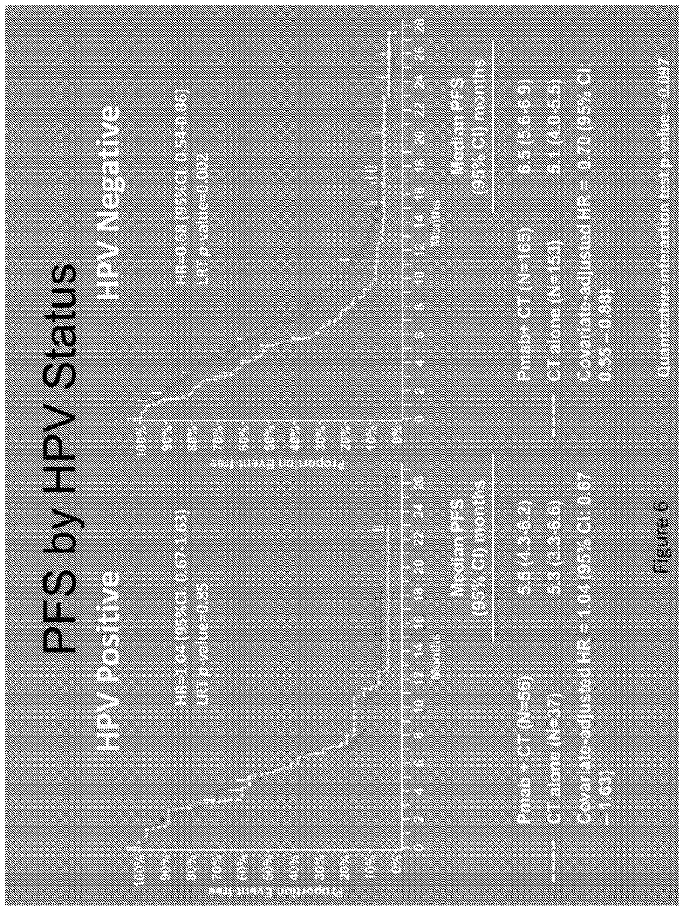

Figure 6 is a plot showing the effect of HPV status on progression-free survival.

### DETAILED DESCRIPTION

The section headings used herein are for organizational purposes only and are not to be construed as limiting the subject matter described.

### Definitions

Unless otherwise defined, scientific and technical terms used in connection with the present invention and disclosure shall have the meanings that are commonly understood by those of ordinary skill in the art. Further, unless otherwise required by context, singular terms shall include pluralities and plural terms shall include the singular.

Generally, nomenclatures utilized in connection with, and techniques of, cell and tissue culture, molecular biology, and protein and oligo- or polynucleotide chemistry and hybridization described herein are those well known and commonly used in the art. Standard techniques are used for recombinant DNA, oligonucleotide synthesis, and tissue culture and transformation (e.g., electroporation, lipofection). Enzymatic reactions, purification and analytical techniques are performed according to the manufacturer's or service provider's specifications or as commonly accomplished in the art or as described herein. The foregoing techniques and procedures are generally performed according to conventional methods well known in the art and as described in various general and more specific references that are cited and discussed throughout the present specification. See, *e.g*., Sambrook et al., Molecular Cloning: A Laboratory Manual (2d ed., Cold Spring Harbor Laboratory Press, Cold Spring Harbor, N.Y. (1989)). The nomenclatures utilized in connection with, and the laboratory procedures and techniques of, analytical chemistry, synthetic organic chemistry, and medicinal and pharmaceutical chemistry described herein are those well known and commonly used in the art. Standard techniques are used for chemical syntheses, chemical analyses, pharmaceutical preparation, formulation, and delivery, and treatment of patients.

Following standard convention, as used herein the terms "a" and "an" mean "one or more" unless context or explicit verbiage dictate otherwise.

In the instant disclosure, the term "or" means "and/or" unless stated otherwise. In the context of a multiple dependent claim, the use of "or" refers back to more than one preceding independent or dependent claim in the alternative only. Furthermore, the use of the term "including", as well as other forms, such as "includes" and "included", is not limiting. Also, terms such as "element" or "component" encompass both elements and components comprising one unit and elements and components that comprise more than one subunit unless specifically stated otherwise.

As used herein, the term "specific binding agent" refers to a natural or non-natural molecule that specifically binds to a target. Examples of specific binding agents include, but are not limited to, proteins, peptides, nucleic acids, carbohydrates, lipids, and small molecule compounds. In certain embodiments, a specific binding agent is an antibody, which can be a human antibody, a humanized antibody or another form of antibody, such as a chimeric antibody. In other embodiments a specific binding agent is a peptibody. In certain embodiments, a specific binding agent is an antigen binding region which can, but need not, be derived from an antibody.

As used herein, the term "agent that specifically binds to an EGFr polypeptide" refers to a specific binding agent that specifically binds any portion of an EGFr polypeptide. In certain embodiments, a specific binding agent to an EGFr polypeptide is an antibody to an EGFr polypeptide, which can be a human antibody, a humanized antibody or another form of a chimeric antibody. In certain embodiments, a specific binding agent to an EGFr polypeptide is an antigen binding region which can, but need not, be derived from an antibody. In certain embodiments, a specific binding agent to an EGFr polypeptide is an antibody to EGFr. In certain embodiments, a specific binding agent to an EGFr polypeptide is Vectibix® (panitumumab) and variants and equivalents thereof. In other examples, a specific binding agent to an EGFr polypeptide is cetuximab (Erbitux®), Iressa^{®} (gefitinib), Tarceva^{®} (erlotinib), Tykerb^{®} (lapatinib), Caprelsa^{®} (vandetanib), zalutumumab (GenMab), nimotuzumab (YM Biosciences), and matuzumab (Merck Serono/Takeda), afatinib (Boehringer-Ingelheim), neratinib (Pfizer), canertinib (PD183805, Pfizer), AP26113 (Ariad), AEE788 (Novartis), BMS-599626 (AC480, Bristol-Myers Squibb), XL-647 (Exelixis), natural EGFr inhibitors such as potato carboxypeptidase inhibitor (PCI) and variants and equivalents of any of these molecules. It will be appreciated that an agent that specifically binds to an EGFr polypeptide, may include dual inhibitors (such as recited herein) that possess specificity to EGFr and at least one additional desired target.

As used herein, the term "specifically binds" refers to the ability of a specific binding agent to bind to a target with greater affinity than it binds to a non-target. In certain embodiments, specific binding refers to binding for a target with an affinity that is at least 10, 50, 100, 250, 500, or 1000 times greater than the affinity for a non-target. In certain embodiments, affinity is determined by an affinity ELISA assay. In certain embodiments, affinity is determined by a BIAcore™ assay. In certain embodiments, affinity is determined by a kinetic method. In certain embodiments, affinity is determined by an equilibrium/solution method. In certain embodiments, an antibody is said to specifically bind an antigen when the dissociation constant between the antibody and one or more of its recognized epitopes is ≤ 1 µM, preferably ≤ 100 nM and most preferably ≤ 10 nM.

As used herein, the term "antibody" refers to both an intact antibody and an antigen binding fragment thereof which competes with the intact antibody for specific binding to a target. An "antigen binding fragment thereof," when used in the context of an antibody, means a portion or fragment of an intact antibody molecule that retains the antigen-binding function. Binding fragments can be produced by recombinant DNA techniques, or by enzymatic or chemical cleavage of intact antibodies such as by cleavage with papain. Binding fragments include Fab, Fab', F(ab')2, Fv, single-chain antibodies ("scFv"), Fd', Fd fragments and fragments comprising a variable region of an antibody. Methods for producing the various fragments from monoclonal antibodies are well known to those skilled in the art (see, *e.g*., Pluckthun, (1992) Immunol. Rev. 130:151-188). In the context of the instant disclosure, an antibody is designated as "substantially inhibiting adhesion of a receptor to a counterreceptor" (*e.g*., EGF to EGFr) when an excess of antibody reduces the quantity of receptor bound to counterreceptor by at least about 20%, 40%, 60%, or 80%, and more preferably at least about 85%, 90%, 95%, 96%, 97%, 98%, or 99% as measured in an *in vitro* competitive binding assay.

An "isolated" antibody or agent that specifically binds to an EGFr polypeptide is an antibody or agent that specifically binds to an EGFr polypeptide that has been identified and separated and/or recovered from a component of the environment in which it is synthesized (*e.g*., a CHO cell). Contaminant components of the environment in which it is synthesized include materials which would interfere with diagnostic or therapeutic uses for the antibody, and may include enzymes, hormones, and other proteinaceous or nonproteinaceous solutes. In preferred embodiments, the antibody will be purified (1) to greater than 95% by weight of antibody as determined by the Lowry method, and terminal or internal amino acid sequencing by use of a spinning cup sequenator; or (2) to homogeneity by SDS-PAGE under reducing or nonreducing conditions using Coomassie blue or, preferably, silver stain. An isolated antibody includes the antibody *in situ* within recombinant cells since at least one component of the antibody's natural environment will not be present. Ordinarily, but not necessarily, an isolated antibody will be prepared by at least one purification step.

An "Fv" or "Fv fragment" of an antibody, including an agent that specifically binds to an EGFr polypeptide, is the minimum fragment of the antibody comprising a complete antigen-recognition and binding site. In a two-chain Fv species, this region comprises a dimer of one heavy- and one light-chain variable domain in tight, noncovalent association. In a single-chain Fv species, one heavy- and one light-chain variable domain can be covalently linked by a flexible peptide linker such that the light and heavy chains can associate in a "dimeric" structure analogous to that in a two-chain Fv species. It is in this configuration that the three CDRs of each variable domain interact to define an antigen-binding site on the surface of the VH-VL dimer. Collectively, the six CDRs confer antigen-binding specificity on the antibody. However, even a single variable domain (or half of an Fv comprising only three CDRs specific for an antigen) has the ability to recognize and bind antigen, although at a lower affinity than the entire binding site.

The term "hypervariable region" of an antibody, including an agent that specifically binds to an EGFr polypeptide, means the amino acid residues of an antibody which are responsible for antigen-binding. The hypervariable region generally comprises amino acid residues from a "complementarity determining region" or "CDR" (*e.g*., residues 24-34 (L1), 50-62 (L2), and 89-97 (L3) in the light chain variable domain and 31-55 (H1), 50-65 (H2) and 95-102 (H3) in the heavy chain variable domain, as defined by Kabat et al., Sequences of Proteins of Immunological Interest, 5th Ed. Public Health Service, National Institutes of Health, Bethesda, MD. (1991)) and/or those residues from a "hypervariable loop" (*e.g*., residues 26-32 (L1), 50-52 (L2) and 91-96 (L3) in the light chain variable domain and 26-32 ((H1), 53-55 (H2) and 96-101 (H3) in the heavy chain variable domain, as defined by Chothia & Lesk J. Mol. Biol 196:901-917 (1987)). "Framework Region" or "FR" residues are those variable domain residues other than the hypervariable region residues as herein defined. It is noted that the residues recited as defining CDRs and hypervariable loop are provided above using the numbering system of Kabat et al. and Chothia & Lesk, these and other systems for defining CDRs and various other features of an antibody (e.g., the AHo system, Honegger & Pluckthun, (2001) J. Mol. Biol. 309:657-70) can be employed interchangeably.

The term "complementarity determining regions" or "CDRs," when used herein, refers to those parts of an antibody, including an agent that specifically binds to an EGFr polypeptide, that make contact with a specific ligand and determine its specificity. The CDRs of antibodies are the most variable part of the protein, giving antibodies their diversity, and are carried on six loops at the distal end of the antibody's variable domains, three loops coming from each of the two variable domains of the antibody.

The term "epitope" includes any protein determinant capable of specific binding to an immunoglobulin and/or T-cell receptor. Epitopic determinants usually consist of chemically active surface groupings of molecules such as amino acids or sugar side chains and usually have specific three dimensional structural characteristics, as well as specific charge characteristics.

The term "agent" is used herein to denote a chemical compound, a mixture of chemical compounds, a biological macromolecule (such as an antibody or other agent that specifically binds to an EGFr polypeptide), or an extract made from biological materials.

As used herein, the terms "label" or "labeled" refers to incorporation of a detectable marker, *e.g.*, by incorporation of a radiolabeled amino acid or by attachment to a polypeptide of biotinyl moieties that can be detected by marked avidin (*e.g*., streptavidin containing a fluorescent marker or enzymatic activity that can be detected by optical or colorimetric methods) or by attachment to an antibody that is specific for a marker to be studied (e.g., p16^{INK4A}). In certain situations, the label or marker can also be therapeutic. Various methods of labeling polypeptides and glycoproteins are known in the art and may be used. Examples of labels for polypeptides include, but are not limited to, the following: radioisotopes or radionuclides (*e.g.*, ³H ¹⁴C, ¹⁵N, ³⁵S, ⁹⁰Y, ⁹⁹Tc ¹¹¹In, ¹²⁵I, ¹³¹I), fluorescent labels (*e.g*., FITC, rhodamine, lanthanide phosphors), enzymatic labels (*e.g*., horseradish peroxidase, β-galactosidase, luciferase, alkaline phosphatase), chemiluminescent groups, biotinyl groups, and predetermined polypeptide epitopes recognized by a secondary reporter (*e.g*., leucine zipper pair sequences, binding sites for secondary antibodies, metal binding domains, epitope tags). In some embodiments, labels are attached by spacer arms of various lengths to reduce potential steric hindrance.

The term "pharmaceutical agent or drug" as used herein refers to a chemical compound or composition capable of inducing a therapeutic effect (preferably a desired therapeutic effect) when properly administered to a patient. Other chemistry terms herein are used according to conventional usage in the art, as exemplified by The McGraw-Hill Dictionary of Chemical Terms (Parker, S., Ed., McGraw-Hill, San Francisco (1985)).

The term "antineoplastic agent" is used herein to refer to agents that have the functional property of inhibiting a development or progression of a neoplasm in a human, particularly a malignant (cancerous) lesion, such as a carcinoma, sarcoma, lymphoma, or leukemia. Inhibition of metastasis is frequently a property of antineoplastic agents. In certain embodiments, an antineoplastic agent is panitumumab.

As used herein, "substantially pure" means an object species is the predominant species present (*i.e.*, on a molar basis it is more abundant than any other individual species in the composition), and preferably a substantially purified fraction is a composition wherein the object species comprises at least about 50 percent (on a molar basis) of all macromolecular species present. Generally, a substantially pure composition will comprise more than about 80 percent of all macromolecular species present in the composition, more preferably more than about 85%, 90%, 95%, 96%, 97%, 98%, or 99%. Most preferably, the object species is purified to essential homogeneity (contaminant species cannot be detected in the composition by conventional detection methods) wherein the composition consists essentially of a single macromolecular species.

As used herein, the term "patient" includes human and animal subjects.

The terms "mammal" and "animal" for purposes of treatment refers to any animal classified as a mammal, including humans, domestic and farm animals, and zoo, sports, or pet animals, such as dogs, horses, cats, cows, etc. Preferably, the mammal is human.

The term "disease state" refers to a physiological state of a cell or of a whole mammal in which an interruption, cessation, or disorder of cellular or body functions, systems, or organs has occurred.

The terms "treat" or "treatment" refer to both therapeutic treatment and prophylactic or preventative measures, wherein the object is to prevent or slow down (lessen) an undesired physiological change or disorder, such as the development or spread of cancer. For purposes of this invention, beneficial or desired clinical results include, but are not limited to, alleviation of symptoms, diminishment of extent of disease, stabilized (*i.e*., not worsening) state of disease, delay or slowing of disease progression, amelioration or palliation of the disease state, and remission (whether partial or total), whether detectable or undetectable. "Treatment" can also mean prolonging survival as compared to expected survival if not receiving treatment. Those in need of treatment include those already with the condition or disorder as well as those prone to have the condition or disorder or those in which the condition or disorder is to be prevented.

The term "responsive" as used herein means that a patient or tumor shows a response after administering an agent, according to the RECIST (Response Evaluation Criteria in Solid Tumors) schedule. The response can be a complete response or a partial response. The term "nonresponsive" as used herein means that a patient or tumor shows stable disease or progressive disease after administering an agent, according to RECIST. RECIST is described, *e.g*., in Therasse et al., (2000) J. Natl. Cancer Inst. 92(3): 205-216. Exemplary agents include specific binding agents to an EGFr polypeptide, including but not limited to, antibodies that specifically bind EGFr.

A "disorder" is any condition that would benefit from one or more treatments. This includes chronic and acute disorders or disease including those pathological conditions which predispose the mammal to the disorder in question. Non-limiting examples of disorders described herein include benign and malignant tumors, leukemias, and lymphoid malignancies. A preferred disorder
is a malignant tumor, including tumors of the oral cavity, pharynx, larynx or hypopharynx.

A "disease or condition related to an EGFr polypeptide" includes one or more of the following: a disease or condition caused by an EGFr polypeptide; a disease or condition contributed to by an EGFr polypeptide; and a disease or condition that is associated with the presence of an EGFr polypeptide. In certain embodiments, a disease or condition related to an EGFr polypeptide is a cancer. Exemplary cancers include, but are not limited to, tumors of the oral cavity, pharynx, larynx or hypopharynx.

In "combined therapy," patients are treated with a specific binding agent for a target antigen in combination with a chemotherapeutic or antineoplastic agent and/or radiation therapy. In certain embodiments, the specific binding agent is panitumumab. Protocol designs will address effectiveness as assessed by reduction in tumor mass as well as the ability to reduce usual doses of standard chemotherapy. These dosage reductions will allow additional and/or prolonged therapy by reducing dose-related toxicity of the chemotherapeutic agent.

"Monotherapy" refers to the treatment of a disorder by administering immunotherapy to patients without an accompanying chemotherapeutic or antineoplastic agent. In certain embodiments, monotherapy comprises administering panitumumab in the absence of a chemotherapeutic or antineoplastic agent and/or radiation therapy.

As used herein, the term "HPV positive" means uniform staining of 10% or more tumor cells in a sample obtained from a subject having a HNSCC tumor in an IHC assay comprising a monoclonal antibody reagent that specifically binds p16^{INK4A}. See, *e.g*., Klussmann et al. (2003) and Belgum et al. (2005). In a specific embodiment context, the term denotes a score of .10 percent positive uniform staining in a formalin fixed paraffin embedded (FFPE) sample obtained from a patient as determined by the CINtec™ Histology kit manufactured and sold by mtm Laboratories of Heidelberg, Germany. A variety of other HPV detection kits are commercially available, as will be appreciated by those of skill in the art.

As used herein, the term "HPV negative" means rare focal staining or difuse staining of less than 10% of the HNSCC tumor cells in a sample obtained from a subject having a HNSCC tumor in an IHC assay comprising a reagent that specifically binds p16^{INK4A}. In a specific embodiment, the term denotes staining of less than 10% in a sample obtained from a patient as determined by the CINtec™ Histology kit manufactured and sold by mtm Laboratories of Heidelberg, Germany.

As used herein, the term "HNSCC tumor" means a squamous cell or basaloid tumor that arises in the head or neck region and includes tumors of the nasal cavity, sinuses, lips, mouth and oral cavity, salivary glands, pharynx, or larynx.

As used herein, the term "EGFr polypeptide" means a polypeptide, or variant thereof, comprising a 170 kDa transmembrane cell surface glycoprotein, triggers a cascade of cellular biochemical events, including EGFr autophosphorylation and internalization, which culminates in cell proliferation (Ullrich et al., Cell 61:203-212, 1990).

As used herein, the term "p16^{INK4A}" means a polypeptide, or variant thereof, comprising the amino acid sequence of Genbank Accession Number GI:4502749.

As used herein, the term "cancer" means an disorder attributable to undesired cell growth, and is characterized by the presence of an undesired tumor. It is noted that the term "cancer" encompasses conditions in which the undesried cell growth has formed a tumor, as well as conditions of undesired cell growth in which a tumor is absent or has not yet developed. The term specifically includes benign and malignant tumors, leukemias, and lymphoid malignancies, in particular breast, rectal, ovarian, stomach, endometrial, salivary gland, kidney, colon, thyroid, pancreatic, prostate or bladder cancer. In certain embodiments a cancer is a disease or condition caused by an EGFr polypeptide, a disease or condition contributed to by an EGFr polypeptide and/or a disease or condition that is associated with the presence of an EGFr polypeptide. In certain embodiments, a disease or condition related to an EGFr polypeptide is a cancer, such as non small cell lung carcinoma, breast, colon, rectum, gastric, brain, bladder, head and neck, ovarian, prostate carcinomas, or a HNSCC tumor, such as a tumor of the larynx, oropharynx, pharynx, or oral cavity.

### Initial Considerations

Disclosed herein are methods directed to a patient having a patient having a tumor, and in a particular aspect, a patient having an HNSCC tumor, notably a locally advanced HNSCC tumor. Such tumors include oropharangeal tumors, tumors of the larynx, tumors of the oral cavity and tumors of the hypopharynx. Such tumors are routinely identified by practitioners in the field of oncology, such as physicians, medical oncologists, histopathologists and oncologic clinicians. Although incidence of these types of tumors is often correlated with tobacco use, particularly cigarette smoking, there is no requirement in the disclosed methods that a patient have used tobacco and therefore any patient having a tumor (such as a HNSCC tumor) that is HPV negative may benefit from the disclosed methods. There is no requirement as to the stage of the patient's tumor; the tumor can be at any stage of growth, for example T2, T3 or T4. The tumor can also exist at any stage of the nodal stage, for example N0, N1, N2a, N2b, N2c or N3. Further, the tumor can be staged as such by any system, for example the AJCC system or the TNM staging system.

As noted herein, the term "patient" refers to any organism having a patient having a tumor, and in a particular aspect, a patient having a locally advanced HNSCC tumor. Preferably the patient is a mammal and more preferably the patient is a human. There is no age restriction on the term "patient," and thus a patient can be an adult male or female, or a male or female child of any age, including infants. Further, a patient can be of any race.

The disclosed methods also relate to therapeutic treatment of such a patient with an agent that specifically binds to an EGFreceptor polypeptide. Any type of agent can be employed, with the proviso that the agent specifically binds to an EGFr polypeptide. Such agents can be biologics, many of which are commonly used in treatment regimens directed to such tumors. A non-limiting list of such agents includes panitumumab (Vectibix®), cetuximab (Erbitux®), Iressa® (gefitinib), Tarceva® (erlotinib), Tykerb® (lapatinib), Caprelsa® (vandetanib), zalutumumab (GenMab), nimotuzumab (YM Biosciences), and matuzumab (Merck Serono/Takeda), afatinib (Boehringer-Ingelheim), neratinib (Pfizer), canertinib (PD183805, Pfizer), AP26113 (Ariad), AEE788 (Novartis), BMS-599626 (AC480, Bristol-Myers Squibb), XL-647 (Exelixis), and natural EGFr inhibitors such as potato carboxypeptidase inhibitor (PCI).

Responsiveness or nonresponsiveness to treatment with a specific binding agent to an EGFr polypeptide can be determined using any established criteria. In a specific example, responsiveness or nonresponsiveness can be determined using the widely adopted RECIST (Response Evaluation Criteria in Solid Tumors) criteria. See, *e.g*., Therasse et al., *supra.* Complete response and partial response according to RECIST are both considered to be responsive to treatment with a specific binding agent to an EGFr polypeptide. Stable disease and progressive disease are both considered to be nonresponsive to treatment with a specific binding agent to an EGFr polypeptide.

All of the disclosed methods can be supplemented as desired. For example, the disclosed methods can optionally additionally comprise making a determination of the responsiveness of a patient having a patient having a tumor, and in a particular aspect, a patient having a locally advanced HNSCC tumor to therapy comprising an agent that specifically binds to an EGFr polypeptide. Such a determination can be made using the RECIST criteria, as described herein.

In another aspect, the disclosed methods can be supplemented by adjusting the therapy of a patient having a patient having a tumor, and in a particular aspect, a patient having a locally advanced HNSCC tumor based on an evaluation of the results of the method. In one embodiment, a patient not receiving therapy comprising an agent that specifically binds to an EGFr polypeptide can be placed on such a regimen, based on the establishment that the patient's tumor is HPV negative. In one embodiment the therapy can comprise administering an agent that specifically binds to an EGFr polypeptide (such as Vectibix®) at a dosage of 6 mg/kg every 14 days as an intravenous infusion over 60 minutes or 90 minutes, depednign on the amount of the agent provided.

In still another aspect, it may be determined that, based on an assessment of the HPV status of the tumor, a patient having a patient having a tumor, and in a particular aspect, a patient having a locally advanced HNSCC tumor receiving therapy comprising an agent that specifically binds to an EGFr polypeptide would benefit from a regimen of therapeutics comprising therapeutics in addition to the patient's current regimen of an agent that specifically binds to an EGFr polypeptide.

It is noted that there are different forms of HNSCC tumors, which are often categorized by the site within the upper aeorodigestive tract that these tumors arise including oropharangeal tumors, larynx tumors, tumors of the oral cavity, and tumors of the hypopharynx. As used herein, the term "HNSCC tumor" encompasses all of these types of tumors. This includes kertininzing, mixed and nonkertinizing morphologic varients Thus, when determining if a locally advanced or recurrent metastatic HNSCC tumor is positive or negative, the determination can be performed on any type of locally advancedor recurrent metastatic HNSCC tumor.

### Method Of Predicting Whether A Patient Having a Tumor, for Example, a HNSCC Tumor, Will Benefit From Treatment Comprising an Agent That Specifically Binds to an EGFr Polypeptide

HPV infection of HNSCC marked by the upregulated expression of the protein p16 ^{ink4A} has been identified as a potential prognostic marker in locally advanced NHSCC (see, *e.g*., Ang et al., (2010) N. Engl. J. Med. 363:24-35). Until the instant disclosure, however, HPV has not been ascribed a treatment response predictive role, particularly in the area of EGFr inhibitor-based therapies. Accordingly, in one aspect of the instant disclosure a method of predicting whether a patient having a tumor, and in a particular aspect, a patient having a HNSCC tumor, will benefit from treatment comprising an agent that specifically binds to an EGFr polypeptide is provided. In one embodiment the method comprises determining whether the patient's tumor is HPV positive or HPV negative, wherein if the patient's tumor is HPV negative the patient is predicted to benefit from treatment with an agent that specifically binds to an EGFr polypeptide.

Initially the patient's tumor is determined to be HPV positive or HPV negative. In order to make the determination, any convenient method can be employed. For example, techniques as varied as IHC, FISH, qPCR or a mass spectrometry-based approach can be employed. Most often, it will be necessary to obtain a sample of the patient's tumor and perform the determination in an *in vitro* setting, for example after preparing the sample for testing using a formalin fixed paraffin embedded (FFPE) sample.

In various embodiments of the disclosed method, the determination of whether a patient's tumor is HPV positive or HPV negative can be made on the basis of an evaluation of any one or combination of HPV markers associated with the tumor. One particularly useful marker is p16^{INK4A}. This marker is indicative of the presence of HPV, and is readily detectable using a variety of approaches. Other markers that can be used to indicate the presence or absence of HPV include HPV E7.

In one specific embodiment, the presence or absence of p16^{INK4A} can be used to determine whether a patient's tumor is HPV positive or negative. If the patient expresses p16^{INK4A}, the patient is designated as HPV positive; if the patient does not express p16^{INK4A}, the patient is designated as HPV negative. The presence or absence of p16^{INK4A} can be readily determined using a commercially available kit or monoclonal or polyclonal antibody of the requiste specificicity. For example, the CINtec® Histology Kit (mtm Laboratories AG, Heidelberg, Germany) can be employed to identify p16^{INK4A} . The CINtec® Histology Kit is an IHC kit designed and labeled for the detection of p16^{INK4A} in the context of cervical cancer. When such a kit is used, it can be validated for use on HNSCC tumors by an independent laboratory. Alternatively, a sample of the patient's tumor can be supplied to a provider which can perform an IHC assay and report the results. In yet another example, an anti-p16^{INK4A} antibody can be generated and used as a component of an IHC procedure.

The determination of whether a patient's tumor is HPV positive or negative based on the pattern and distribution of p16^{ink4A} protein can be made on the basis of scoring guidelines. The guidelines can be quantitative, semi-quantitative or qualitative. In one example, when p16^{INK4A} is used as a marker and the CINtec® Histology Kit is employed to determine the presence of the marker, the CINtec® Staining Atlas (CINtec® p^{16INK4A} Staining Atlas, Trunk et al., mtm Laboratories AG, Heidleberg, Germany) can be used to identify HPV positive and HPV negative tumors. Alternatively, a set of scoring guidelines can be established using p16^{ink4a} staining patterns as observed in a reference population of SCCHN evaluated traditional histological methodologies or molecular probes for the espression mRNA transcribed from the HPV genes E6 and E7.

As demonstrated by the data presented in the Examples, patients whose tumor was HPV negative and who received therapy comprising an agent that specifically binds to an EGFr polypeptide showed an enhancement in progression-free survival and in overall survival. Thus, if the patient's tumor (*e.g*., a HNSCC tumor) is HPV negative the patient is predicted to benefit from treatment with an agent that specifically binds to an EGFr polypeptide.

### Method of Prolonging the Overall Survival of a Patient Having a Tumor, for Example a HNSCC Tumor

As demonstrated by the data presented in the Examples provided herein, patients whose tumor was HPV negative and who received therapy comprising an agent that specifically binds to an EGFr polypeptide showed an enhancement in overall survival, as well as in progression-free survival. Accordingly, in another aspect of the instant disclosure a method of prolonging the overall survival of a patient having a a tumor, and in a particular aspect, a patient having locally advanced HNSCC or recurrent or metastatic tumor is provided. In one embodiment, the method comprises determining whether the patient's tumor is HPV positive or HPV negative, and administering an agent that specifically binds to an EGFr polypeptide to the patient if the patient's tumor is HPV negative, whereby the overall survival and/or progression-free survival of the patient is prolonged.

When performing the method, the patient's tumor status is determined to be HPV positive or HPV negative. As is the case with all of the disclosed methods, in order to make the determination, any convenient method known to those skilled in the art can be employed. For example, techniques as varied as IHC, FISH, qPCR or a mass spectrometry-based approach can be employed. Most often, it will be desirable to obtain a sample of the patient's tumor and perform the determination in an in vitro setting.

In various embodiments of the disclosed method, the determination of whether a patient's tumor is HPV positive or HPV negative can be made on the basis of an evaluation of any one or combination of HPV markers associated with the tumor. One particularly useful marker is p16^{INK4A}. This marker is indicative of the presence of HPV, and the expression of two described viral oncogenes E6 and E7 and is readily detectable using a variety of approaches. Other markers that can be used to indicate the presence or absence of HPV include HPV E7.

In one specific embodiment, the presence or absence of p16^{INK4A} can be used to determine whether a patient's tumor is HPV positive or negative. If the patient expresses p16^{INK4A}, with the requiste degree and distribution of staining of identifiable tumor cells the patient is designated as HPV positive; if the patient does not express p16^{INK4A} the patient is designated as HPV negative. The presence or absence of p16^{INK4A} can be readily determined using a commercially available kit or a service provider. For example, the CINtec® Histology Kit (mtm Laboratories AG, Heidelberg, Germany) can be employed to idetntify p16^{INK4A}. The CINtec® Histology Kit is an IHC kit designed for the detection of p16^{INK4A} in the context of cervical cancer. When such a kit is used, it can be validated by an independent laboratory. Alternatively, a sample of the patient's tumor can be supplied to a provider which can perform an IHC assay and report the results. In yet another example, an anti-p16^{INK4A} antibody can be generated and used as a component of an IHC procedure.

The determination of whether a patient's tumor is HPV positive or negative can be made on the basis of scoring guidelines. The guidelines can be quantitative, semi-quantitative or qualitative. In one example, when p16^{INK4A} is used as a marker and the CINtec® Histology Kit is employed to determine the presence of the marker, the CINtec® Staining Atlas (CINtec® p16^{INK4A} Staining Atlas, Trunk et al., mtm Laboratories AG, Heidleberg, Germany) can be used to identify HPV positive and HPV negative tumors. Alternatively, a set of scoring guidelines can be established using traditional histological methodologies.

Continuing, if the tumor is HPV negative, an agent that specifically binds to an EGFr polypeptide is administered to the patient. As noted herein, the data presented in the Examples indicates that when a patient's tumor is HPV negative and an agent that specifically binds to an EGFr polypeptide is administered, the overall and/or progression-free survival of the patient is prolonged.

### Method of Stratifying a Population of Patients Having a Tumor, for Example a HNSCC Tumor

As demonstrated by the data presented in the Examples, patients having a a tumor, and in a particular aspect, a patient having locally advanced or recurrent metastatic HNSCC tumor that is HPV negative will benefit from a therapy comprising an agent that specifically binds to EGFr. Accordingly, it can be desirable to identify or stratify such patients for treatment with an agent that specifically binds to an EGFr polypeptide, using HPV status as an indicator. Thus, in another aspect of the current disclosure a method of stratifying a population of patients having a locally advanced HNSCC tumor into groups that will benefit and those that will benefit more from therapy comprising an agent that specifically binds to an EGFr polypeptide is provided.

When performing the method, the patient's tumor status is determined to be HPV positive or HPV negative. As is the case will all of the disclosed methods, in order to make the determination, any convenient method can be employed. For example, techniques as varied as IHC, FISH, qPCR or a mass spectrometry-based approach can be employed. Most often, it will be desirable to obtain a sample of the patient's tumor and perform the determination in an *in vitro* setting.

In various embodiments, the determination of whether a patient's tumor is HPV positive or HPV negative can be made on the basis of an evaluation of any one or combination of HPV markers associated with the tumor. One particularly useful marker is p16^{INK4A}. This marker is indicative of the presence of HPV, and is readily detectable using a variety of approaches. Other markers that can be used to indicate the presence or absence of HPV include HPV E7.

In one specific embodiment, the presence or absence of p16^{INK4A} can be used to determine whether a patient's tumor is HPV positive or negative. If the patient expresses p16^{INK4A}, the patient is designated as HPV positive; if the patient does not express p16^{INK4A}, the patient is designated as HPV negative. The presence or absence of p16^{INK4A} can be readily determined using a commercially available kit or a service provider. For example, the CINtec® Histology Kit (mtm Laboratories AG, Heidelberg, Germany) can be employed to identify p16^{INK4A}. The CINtec® Histology Kit is an IHC kit designed for the detection of p16^{INK4A} in the context of cervical cancer. When such a kit is used, it can be validated by an independent laboratory. Alternatively, a sample of the patient's tumor can be supplied to a provider which can perform an IHC assay and report the results. In yet another example, an anti-p16^{INK4A} antibody can be generated and used as a component of an IHC procedure.

The determination of whether a patient's tumor is HPV positive or negative can be made on the basis of scoring guidelines. The guidelines can be quantitative, semi-quantitative or qualitative. In one example, when p16^{INK4A} is used as a marker and the CINtec® Histology Kit is employed to determine the presence of the marker, the CINtec® Staining Atlas (CINtec p16^{INK4A} Staining Atlas, Trunk et al., mtm Laboratories AG, Heidleberg, Germany) can be used to identify HPV positive and HPV negative tumors. Alternatively, a set of scoring guidelines can be established using traditional histological methodologies.

Continuing, patients whose tumors are HPV negative are selected for treatment with a therapy comprising an agent that specifically binds to an EGFr polypeptide. These patients are expected to benefit more than patients who are HPV positive in a therapy comprising an agent that specifically binds to an EGFr polypeptide. By stratifying a group of patients having a a tumor, and in a particular aspect, a patient having locally advanced HNSCC tumor, a medical professional will be able to tailor a therapy to the patient's particular needs and enhances the likelihood that the patient will respond positively.

### Method of Treating a Patient Having a Tumor, for Example a Locally Advanced HNSCC Tumor

As described herein and in the Examples, it has been determined that patients having a a tumor, and in a particular aspect, a patient having locally advanced HNSCC tumor that is HPV negative exhibit an enhancement in overall and/or progression-free survival when treated with an agent that specifically binds to an EGFr polypeptide. Accordingly, a method of treating such patients is provided. In one embodiment of a method of treating a patient having a locally advanced HNSCC tumor comprises determining the patient's tumor status to be HPV positive or HPV negative. As is the case will all of the disclosed methods, in order to make the determination, any convenient method can be employed. For example, techniques as varied as IHC, FISH, qPCR or a mass spectrometry-based approach can be employed. Most often, it will be desirable to obtain a sample of the patient's tumor and perform the determination in an *in vitro* setting.

In various embodiments, the determination of whether a patient's tumor is HPV positive or HPV negative can be made on the basis of an evaluation of any one or combination of HPV markers associated with the tumor. One particularly useful marker is p16^{INK4A}. This marker is indicative of the presence of HPV, and is readily detectable using a variety of approaches. Other markers that can be used to indicate the presence or absence of HPV include HPV E7.

In one specific embodiment, the presence or absence of p16^{INK4A} can be used to determine whether a patient's tumor is HPV positive or negative. If the patient expresses p16^{INK4A}, the patient is designated as HPV positive; if the patient does not express p16^{INK4A}, the patient is designated as HPV negative. The presence or absence of p16^{INK4A} can be readily determined using a commercially available kit or a service provider. For example, the CINtec® Histology Kit (mtm Laboratories AG, Heidelberg, Germany) can be employed to idetntify p16^{INK4A}. The CINtec® Histology Kit is an IHC kit designed for the detection of p16^{INK4A} in the context of cervical cancer. Alternatively, a sample of the patient's tumor can be supplied to a provider which can perform an IHC assay and report the results. In yet another example, an anti-p16^{INK4A} antibody can be generated and used as a component of an IHC procedure.

The determination of whether a patient's tumor is HPV positive or negative can be made on the basis of scoring guidelines. The guidelines can be quantitative, semi-quantitative or qualitative. In one example, when p16^{INK4A} is used as a marker and the CINtec® Histology Kit is employed to determine the presence of the marker, the CINtec® Staining Atlas (CINtec® p16^{INK4A} Staining Atlas, Trunk et al., mtm Laboratories AG, Heidleberg, Germany) can be used to identify HPV positive and HPV negative tumors. Alternatively, a set of scoring guidelines can be established using traditional histological methodologies.

Continuting with the method, if the patient's tumor is HPV negative, the patient is administered an agent that specifically binds to an EGFr polypeptide. As shown in the data presented herein, patients having tumors that are HPV negative show enhancements in overall survival and/or in progression-free survival when administered an agent that specifically binds to an EGFr polypeptide. By performing the disclosed method, the medical professional can provide a more efficacious treatment regimen to patients suffering from this condition.

The method can further comprise a treatment regimen known to be effective for the particular agent that specifically binds to an EGFr polypeptide.

### EXAMPLES

The following examples, including the experiments conducted and results achieved are provided for illustrative purpose only and are not to be construed as limiting upon the claims. Additional embodiments of the disclosed methods will be apparent to those skilled in the art after considering the instant disclosure and the following Examples. Accordingly, it is intended that the instant disclosure, including the following Examples, be considered as providing particular, but non-limiting, embodiments of the disclosed methods.

### Example 1

### Identification of HPV Positive/Negative Status

In order to identify HPV status of the subjects taking part in the trial, HNSCC tumor samples were acquired from subjects participating in a HNSCC clinical trial involving systemic cytotoxic chemotherapy with and without panitumumab Of the 657 subjects in the trial, 67% or 411 subjects provided evaluable archival FFPE tumor samples containing at least 10 percent tumor cells samples..

The demographics and disease characteristics of patients that participated in the trial is summarized in Table 1:

**Table 1**

| **Demographics and Disease Characteristics of Patients** | | | | | | |
|---|---|---|---|---|---|---|
| | **ITT** | | **HPV+** | | **HPV-** | |
| | **Pmab + CT (n = 327)** | **CT (n = 330)** | **Pmab + CT (n = 56)** | **CT (n = 37)** | **Pmab + CT (n = 165)** | **CT (n = 153)** |
| **Sex, male - %** | **87** | **87** | **84** | **81** | **87** | **89** |
| **Race, white - %** | **82** | **82** | **80** | **86** | **85** | **84** |
| **Age, median- yrs** | **58** | **59** | **58** | **60** | **57** | **59** |
| **ECOG 0 - %** | **30** | **30** | **38** | **38** | **33** | **26** |
| **£ 10 pack-yrs - %** | **26** | **22** | **43** | **24** | **19** | **15** |
| **>10 pack-yrs - %** | **61** | **65** | **52** | **57** | **64** | **71** |
| **Prior platinum - %** | **39** | **34** | **38** | **54** | **40** | **31** |

| | **ITT** | | **HPV+** | | **HPV-** | |
|---|---|---|---|---|---|---|
| | **Pmab + CT (n = 327)** | **CT (n = 330)** | **Pmab + CT (n = 56)** | **CT (n = 37)** | **Pmab + CT (n = 165)** | **CT (n = 153)** |
| **Wt loss ≤ 5% - %** | **81** | **79** | **80** | **81** | **82** | **80** |
| **Wt loss > 5% - %** | **18** | **21** | **18** | **19** | **18** | **20** |
| **Region - %** | | | | | | |
| **N/S America** | **15** | **17** | **23** | **32** | **15** | **18** |
| **W Europe** | **31** | **35** | **32** | **41** | **34** | **38** |
| **Asia Pacific** | **17** | **13** | **16** | **5** | **16** | **10** |
| **E Europe** | **38** | **35** | **29** | **22** | **35** | **33** |

Tumor specimens were sent to an independent laboratory for pathology evaluation and tumor microarray construction. A single pathologist, with significant experience reading squamous cell carcinoma of the head and neck, examined a standard a hemotoxylin and eosin stained 5 micron tissue section of every submitted sample. The pathologic review included: Diagnosis (presence or absence of HNSCC); Specimen type (tumor resection/lymph node/metastases (e.g., lung, liver)); Histological type (HNSCC (NOS) versus Papillary versus Spindle cell (sarcomatoid) versus Basaloid versus other, including adenosquamous; Differentiation status (well, moderate, poorly, undifferentiated or unable to determine); Tumor Borders (infiltrating versus pushing); Inflammatory response; Necrosis, including comedo necrosis; and other observations, as applicable. As a result of this analysis, it was determined that of the of the 657 subjects in the trial, 67% had samples with greater than 10% viable tumor. A tumor microarray set containing 1083 indivdual 1 mm cores was created to facilitate the standardization of the IHC assay and to provide multiple replicates of samples where tumor tissue permitted

HPV status was measured using the commercially available CINtec™ Histology kit (mtm Laboratories, Heidelberg, Germany). The CINtec™ Histology kit is a semi-quantitative, immunocytochemical assay for the evaluation of overexpressed cyclin-dependent kinase inhibitor, p16^{INK4A} protein, in formalin fixed, paraffin-embedded tissue sections. The presence or absence of p16^{INK4A} protein is indicative that the sample is HPV positive or HPV negative. The antibody clone is E6H4. The commercially available kit is FDA cleared and labeled for use in cervical cancer tissue.

More specifically, subject tumor specimens were scored positive, negative or failed according to a prespecified IHC scoring guideline. Essentially, a subject was determined to have a HPV positive tumor (HPV+) when uniform p16^{INK4A} protein expression was detected in at least 10% of tumor cells. A subject was determined to have a HPV negative tumor (HPV-) when p16^{INK4A} protein was not present or was observed in less than 10% of tumor cells.

Prior to using the assay on samples acquired from subjects in the clinical trial, a verification of the CINtec™ Histology kit's performance in formalin fixed paraffin embedded (FFPE) HNSCC tumor specimens was performed according to Clinical Laboratory Improvement Amendments (CLIA) regulations. An independent testing laboratory performed an analytical validation of the CINtec™ Histology kit for use on HNSCC and provided a validation package. In addition an immunohistochemical scoring guideline was developed and adhered to during sample testing. Samples were dichotomously scored as positive or negative based on the results of the validated assay.

Analysis of the samples for the presence or absence of HPV provided the data presented in Table 2:

**Table 2**

| **Results of HPV Analysis** | | | | | |
|---|---|---|---|---|---|
| Tissue Site | All Tumors N=657 | HPV Evaluable N=377 (57%) | HPV Positive N=83 | HPV Negative N=294 | HPV Unevaluable N=280 (43%) |
| Hypopharynx | 13% | 14% | 8% | 16% | 13% |
| Larynx | 30% | 32% | 28% | 33% | 27% |
| Oral Cavity | 29% | 27% | 19% | 29% | 32% |
| Oropharynx | 27% | 28% | 45% | 23% | 28% |

### Example 2

### Effect of HPV Status on Panitumumab Therapy on Overall Survival

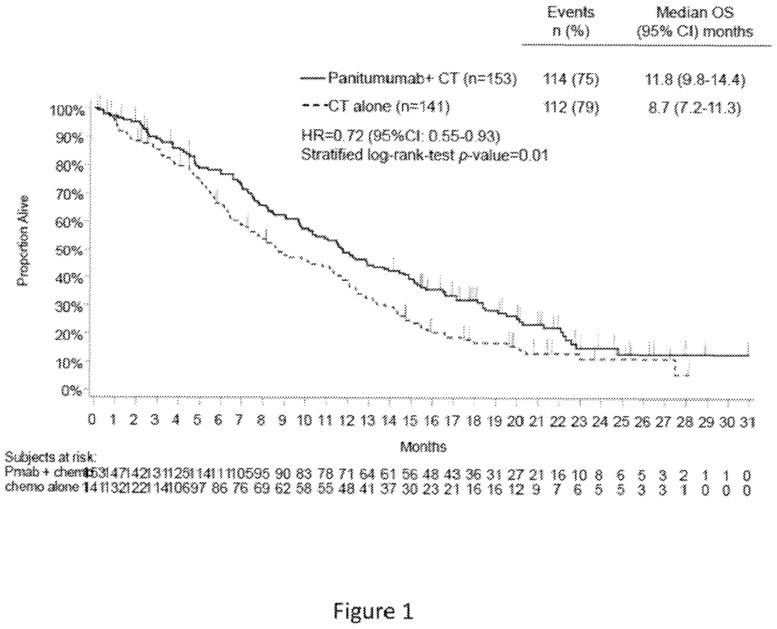

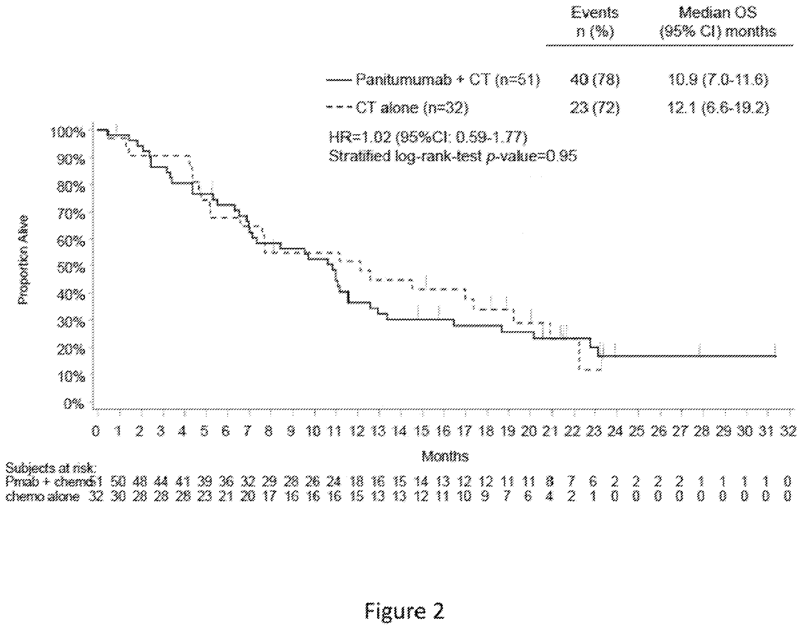

The overall survival of HPV positive and HPV negative subjects in the trial was examined. Figures 1 and 2 are Kaplan-Meier survival curves summarizing the results of the study. A comparison of Figures 1 and 2 shows that subjects whose HNSCC tumors were HPV negative that were treated with panitumumab and chemotherapy showed an enhancement in overall survival over those subjects whose HNSCC tumors were HPV positive.

More particularly, those subjects whose tumors were HPV positive and were treated with panitumumab and chemotherapy showed a median overall survival of 10.9 months, while those subjects whose tumors were HPV negative and were treated with panitumumab and chemotherapy showed a median overall survival of 11.8 months. It is also noted that subjects whose tumors were HPV negative and were treated with panitumumab and chemotherapy showed an enhancement in median overall survival of 3.1 months over those subjects who received chemotherapy alone.

Independent prognostic factors for overall survival are summarized in Table 3:

**Table 3**

| **Independent Prognostic Factors for Overall Survival** | | | |
|---|---|---|---|
| | **Factor** | **HR** | **P-value** |
| **HPV Negative** | **ECOG (0 vs 1/2)** | **0.66** | **0.004** |
| | **Previously CT or RT (yes vs no)** | **1.345** | **0.078** |
| | **Prior platin (yes vs no)** | **1.246** | **0.097** |
| **HPV Positive** | **ECOG (0 vs 1/2)** | **0.567** | **0.03** |
| | **Pack-years (>10 vs <=10)** | **1.963** | **0.011** |
| | **>5% invol weight loss last 6 mos (yes vs no)** | **2.542** | **0.002** |
| | **Prior platin (yes vs no)** | **1.498** | **0.096** |

### Example 3

### Effect of HPV Status on Panitumumab Therapy on Progression-free Survival

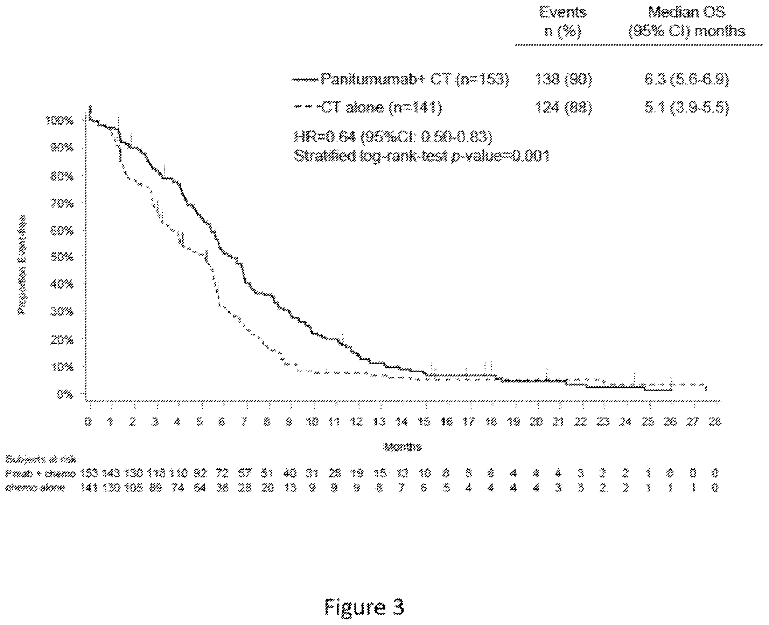

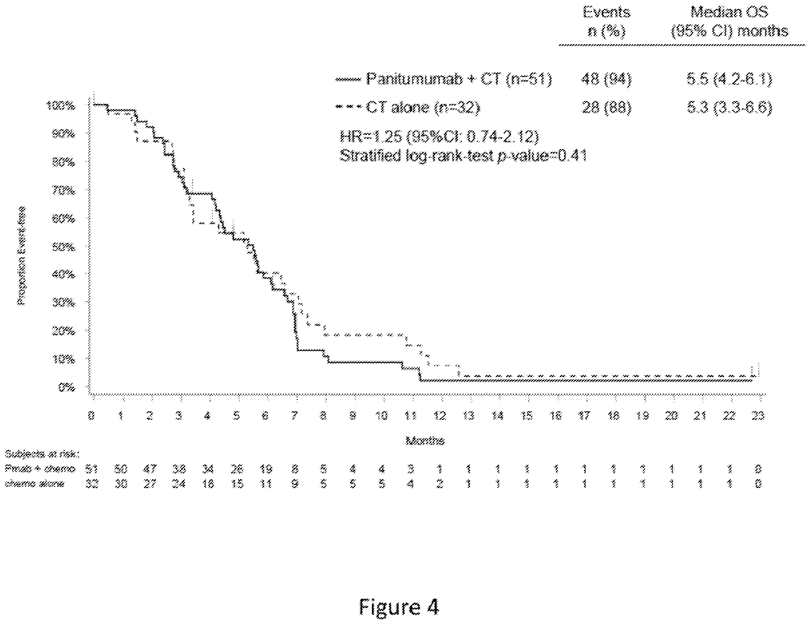

The overall survival of HPV positive and HPV negative subjects in the trial was examined. Figures 3 and 4 are Kaplan-Meier survival curves summarizing the results of the study. A comparison of Figures 3 and 4 shows that subjects whose HNSCC tumors were HPV negative that were treated with panitumumab and chemotherapy showed an enhancement in progression-free survival over those subjects whose HNSCC tumors were HPV positive.

More particularly, those subjects whose tumors were HPV positive and were treated with panitumumab and chemotherapy showed a median progression-free survival of 5.5 months, while those subjects whose tumors were HPV negative and were treated with panitumumab and chemotherapy showed a median overall survival of 6.3 months. It is also noted that subjects whose tumors were HPV negative and were treated with panitumumab and chemotherapy showed an enhancement in median overall survival of 1.2 months over those subjects who received chemotherapy alone.

### Summary of Examples 1-3

HPV status was determined in 57% of the 657 subjects enrolled in the trial. It was determined that 21% of the HNSCC subjects for which HPV status was determined were found to be HPV positive. The data shows that treatment with panitumumab and chemotherapy improved overall survival in subjects whose tumors were determined to be HPV negative over those subjects whose tumors were HPV positive by a median of 0.9 months. The data shows that treatment with panitumumab and chemotherapy improved progression-free survival in subjects whose tumors were determined to be HPV negative over those subjects whose tumors were HPV positive by a median of 0.8 months.

## Claims

1. A method of predicting whether a patient having a tumor will benefit from treatment comprising an agent that specifically binds to an EGFr polypeptide, comprising determining whether the patient's tumor is HPV positive or HPV negative, wherein if the patient's tumor is HPV negative, the patient is predicted to benefit from treatment with an agent that specifically binds to an EGFr polypeptide, wherein the agent that specifically binds to an EGFr polypeptide is an antibody to EGFr.

2. The method of claim 1, wherein the determining comprises determining the presence or absence of p16^{INK4A} in a tumor sample obtained from the patient, wherein the presence of p16^{INK4A} indicates that the patient is HPV positive and the absence of p16^{INK4A} indicates that the patient is HPV negative.

3. The method of claim 1, wherein the antibody to EGFr is panitumumab.

4. The method of claim 2, wherein the determining the presence or absence of p16^{INK4A} in a tumor comprises performing an immunohistochemistry (IHC) assay to identify the presence or absence of p16^{INK4A} in a sample of the tumor.

5. The method of claim 1, wherein the tumor is a locally advanced tumor or a recurrent metastatic tumor; preferably wherein the tumor is selected from the group consisting of an oropharangeal tumor, a larynx tumor, a tumor of the oral cavity and a tumor of the hypopharynx.

6. A monoclonal antibody that specifically binds to an EGFr polypeptide for use in a method of prolonging the progression-free and/or overall survival of a patient having a tumor that has been determined to be HPV negative, wherein the tumor is determined to be HPV negative by determining the presence or absence of p16^{INK4A} in a tumor obtained from the patient, wherein the absence of p16^{INK4A} indicates that the patient is HPV negative.

7. A monoclonal antibody that specifically binds to an EGFr polypeptide for use in the treatment of a patient having a tumor that has been determined to be HPV negative, wherein the tumor is determined to be HPV negative by determining the presence or absence of p16^{INK4A} in a tumor obtained from the patient, wherein the absence of p16^{INK4A} indicates that the patient is HPV negative.

8. The antibody that specifically binds to an EGFr polypeptide for the use according to claim 6 or 7, wherein the antibody to EGFr is panitumumab.

9. The antibody that specifically binds to an EGFr polypeptide for the use according to claim 6 or 7, wherein the determining the presence or absence of p16^{INK4A} in a tumor comprises performing an immunohistochemistry (IHC) assay to identify the presence or absence of p16^{INK4A} in a sample of the tumor.

10. The antibody that specifically binds to an EGFr polypeptide for the use according to claim 6 or 7, wherein the tumor is a locally advanced tumor or a recurrent metastatic tumor; preferably wherein the tumor is selected from the group consisting of an oropharangeal tumor, a larynx tumor, a tumor of the oral cavity and a tumor of the hypopharynx.

11. The antibody that specifically binds to an EGFr polypeptide for the use according to claim 6 or 7, wherein chemotherapy is used in addition to the antibody that specifically binds to an EGFr polypeptide.

12. A method of stratifying a population of patients having a tumor comprising:
(a) determining whether the patient's tumor is HPV positive or HPV negative; and
(b) selecting patients whose tumors are HPV negative for treatment with a therapy comprising an agent that specifically binds to an EGFr polypeptide.

13. The method of claim 12, wherein the determining comprises determining the presence or absence of p16^{INK4A} in a tumor obtained from the patient, wherein the presence of p16^{INK4A} indicates that the patient is HPV positive and the absence of p16^{INK4A} indicates that the patient is HPV negative.

14. The method of claim 12, wherein the agent that specifically binds to an EGFr polypeptide is an antibody to EGFr; preferably wherein the antibody to EGFr is panitumumab.

15. The method of claim 12, wherein the determining the presence or absence of p16^{INK4A} in a tumor comprises performing an immunohistochemistry (IHC) assay to identify the presence or absence of p16^{INK4A} in a sample of the tumor.

16. The method of claim 12, wherein the tumor is a locally advanced tumor or a recurrent metastatic tumor; preferably wherein the tumor is selected from the group consisting of an oropharangeal tumor, a larynx tumor, a tumor of the oral cavity and a tumor of the hypopharynx.

## Patentansprüche

1. Verfahren zum Prognostizieren, ob ein Patient, der einen Tumor hat, von einer Behandlung, die ein Agens umfasst, welches spezifisch an ein EGFr-Polypeptid bindet, profitieren wird, umfassend das Bestimmen, ob der Tumor des Patienten HPV-positiv oder HPV-negativ ist, wobei prognostiziert wird, dass der Patient, wenn der Tumor des Patienten HPV-negativ ist, von einer Behandlung mit einem Agens, das spezifisch an ein EGFr-Polypeptid bindet, profitieren wird, wobei es sich bei dem Agens, das spezifisch an ein EGFr-Polypeptid bindet, um einen Antikörper gegen EGFr handelt.

2. Verfahren nach Anspruch 1, wobei das Bestimmen es umfasst, das Vorliegen oder Fehlen von p16^{INK4A} in einer vom Patienten gewonnenen Tumorprobe zu ermitteln, wobei das Vorliegen von p16^{INK4A} anzeigt, dass der Patient HPV-positiv ist, und das Fehlen von p16^{INK4A} anzeigt, dass der Patient HPV-negativ ist.

3. Verfahren nach Anspruch 1, wobei es sich bei dem Antikörper gegen EGFr um Panitumumab handelt.

4. Verfahren nach Anspruch 2, wobei das Ermitteln des Vorliegens oder Fehlens von p16^{INK4A} in einem Tumor es umfasst, einen immunhistochemischen (IHC) Test vorzunehmen, um das Vorliegen oder Fehlen von p16^{INK4A} in einer Probe des Tumors zu bestimmen.

5. Verfahren nach Anspruch 1, wobei es sich bei dem Tumor um einen lokal fortgeschrittenen Tumor oder um einen rezidivierenden, metastasierenden Tumor handelt, wobei der Tumor vorzugsweise aus der Gruppe, die aus einem oropharyngealen Tumor, einem Larynxtumor, einem Tumor der Mundhöhle und einem Tumor des Hypopharynx besteht, ausgewählt ist.

6. Monoklonaler Antikörper, der spezifisch an ein EGFr-Polypeptid bindet, zur Verwendung in einem Verfahren zum Verlängern des progressionsfreien und/oder gesamten Überlebens eines Patienten, der einen Tumor hat, der als HPV-negativ bestimmt wurde, wobei der Tumor durch Ermitteln des Vorliegens oder Fehlens von p16^{INK4A} in einem vom Patienten gewonnenen Tumor als HPV-negativ bestimmt wird, wobei das Fehlen von p16^{INK4A} anzeigt, dass der Patient HPV-negativ ist.

7. Monoklonaler Antikörper, der spezifisch an ein EGFr-Polypeptid bindet, zur Verwendung in der Behandlung eines Patienten, der einen Tumor hat, der als HPV-negativ bestimmt wurde, wobei der Tumor durch Ermitteln des Vorliegens oder Fehlens von p16^{INK4A} in einem vom Patienten gewonnenen Tumor als HPV-negativ bestimmt wird, wobei das Fehlen von p16^{INK4A} anzeigt, dass der Patient HPV-negativ ist.

8. Antikörper, der spezifisch an ein EGFr-Polypeptid bindet, zur Verwendung gemäß Anspruch 6 oder 7, wobei es sich bei dem Antikörper gegen EGFr um Panitumumab handelt.

9. Antikörper, der spezifisch an ein EGFr-Polypeptid bindet, zur Verwendung gemäß Anspruch 6 oder 7, wobei das Ermitteln des Vorliegens oder Fehlens von p16^{INK4A} in einem Tumor es umfasst, einen immunhistochemischen (IHC) Test vorzunehmen, um das Vorliegen oder Fehlen von p16^{INK4A} in einer Probe des Tumors zu bestimmen.

10. Antikörper, der spezifisch an ein EGFr-Polypeptid bindet, zur Verwendung gemäß Anspruch 6 oder 7, wobei es sich bei dem Tumor um einen lokal fortgeschrittenen Tumor oder um einen rezidivierenden, metastasierenden Tumor handelt, wobei der Tumor vorzugsweise aus der Gruppe, die aus einem oropharyngealen Tumor, einem Larynxtumor, einem Tumor der Mundhöhle und einem Tumor des Hypopharynx besteht, ausgewählt ist.

11. Antikörper, der spezifisch an ein EGFr-Polypeptid bindet, zur Verwendung gemäß Anspruch 6 oder 7, wobei zusätzlich zum Antikörper, der spezifisch an ein EGFr-Polypeptid bindet, Chemotherapie eingesetzt wird.

12. Verfahren zum Stratifizieren einer Population von Patienten, die einen Tumor haben, umfassend:
(a) das Bestimmen, ob der Tumor des Patienten HPV-positiv oder HPV-negativ ist, und
(b) das Selektieren von Patienten, deren Tumoren HPV-negativ sind, für eine Behandlung mit einer Therapie, die ein Agens, das spezifisch an ein EGFr-Polypeptid bindet, umfasst.

13. Verfahren nach Anspruch 12, wobei das Bestimmen es umfasst, das Vorliegen oder Fehlen von p16^{INK4A} in einem vom Patienten gewonnenen Tumor zu ermitteln, wobei das Vorliegen von p16^{INK4A} anzeigt, dass der Patient HPV-positiv ist, und das Fehlen von p16^{INK4A} anzeigt, dass der Patient HPV-negativ ist.

14. Verfahren nach Anspruch 12, wobei es sich bei dem Agens, das spezifisch an ein EGFr-Polypeptid bindet, um einen Antikörper gegen EGFr handelt, wobei es sich vorzugsweise bei dem Antikörper gegen EGFr um Panitumumab handelt.

15. Verfahren nach Anspruch 12, wobei das Ermitteln des Vorliegens oder Fehlens von p16^{INK4A} in einem Tumor es umfasst, einen immunhistochemischen (IHC) Test vorzunehmen, um das Vorliegen oder Fehlen von p16^{INK4A} in einer Probe des Tumors zu bestimmen.

16. Verfahren nach Anspruch 12, wobei es sich bei dem Tumor um einen lokal fortgeschrittenen Tumor oder um einen rezidivierenden, metastasierenden Tumor handelt, wobei der Tumor vorzugsweise aus der Gruppe, die aus einem oropharyngealen Tumor, einem Larynxtumor, einem Tumor der Mundhöhle und einem Tumor des Hypopharynx besteht, ausgewählt ist.

## Revendications

1. Procédé pour prédire si un traitement comprenant un agent qui se lie spécifiquement à un polypeptide EGFr sera bénéfique pour un patient atteint d'une tumeur, comprenant le fait de déterminer si la tumeur du patient est positive à HPV ou négative à HPV, dans lequel, si la tumeur du patient est négative à HPV, on prédit qu'un traitement avec un agent se liant spécifiquement à un polypeptide EGFr sera bénéfique pour le patient, dans lequel l'agent qui se lie spécifiquement à un polypeptide EGFr est un anticorps du EGFr.

2. Procédé selon la revendication 1, dans lequel la détermination comprend le fait de déterminer la présence ou l'absence de p16^{INK4A} dans un échantillon de tumeur prélevé sur le patient, dans lequel la présence de p16^{INK4A} indique que le patient est positif à HPV et l'absence de p16^{INK4A} indique que le patient est négatif à HPV.

3. Procédé selon la revendication 1, dans lequel l'anticorps du EGFr est un panitumumab.

4. Procédé selon la revendication 2, dans lequel la détermination de la présence ou de l'absence de p16^{INK4A} dans une tumeur comprend la réalisation d'un examen immunohistochimique (IHC) pour identifier la présence ou l'absence de p16^{INK4A} dans un échantillon de la tumeur.

5. Procédé selon la revendication 1, dans lequel la tumeur est une tumeur localement avancée ou une tumeur métastatique récidivante, de préférence dans lequel la tumeur est sélectionnée dans le groupe comprenant une tumeur de l'oropharynx, une tumeur du larynx, une tumeur de la cavité buccale et une tumeur de l'hypopharynx.

6. Anticorps monoclonal se liant spécifiquement à un polypeptide EGFr, destiné à être utilisé dans un procédé d'allongement de la survie progression-libre et/ou globale d'un patient atteint d'une tumeur dont on a déterminé qu'elle est négative à HPV, dans lequel on détermine que la tumeur est négative à HPV en déterminant la présence ou l'absence de p16^{INK4A} dans une tumeur prélevée sur le patient, dans lequel l'absence de p16^{INK4A} indique que le patient est négatif à HPV.

7. Anticorps monoclonal se liant spécifiquement à un polypeptide EGFr, destiné à être utilisé dans le traitement d'un patient atteint d'une tumeur dont on a déterminé qu'elle est négative à HPV, dans lequel on détermine que la tumeur est négative à HPV en déterminant la présence ou l'absence de p16^{INK4A} dans une tumeur prélevée sur le patient, dans lequel l'absence de p16^{INK4A} indique que le patient est négatif à HPV.

8. Anticorps se liant spécifiquement à un polypeptide EGFr, pour l'utilisation selon la revendication 6 ou 7, dans lequel l'anticorps du EGFr est un panitumumab.

9. Anticorps qui se lie spécifiquement à un polypeptide EGFr, pour l'utilisation selon la revendication 6 ou 7, dans lequel la détermination de la présence ou de l'absence de p16^{INK4A} dans une tumeur comprend la réalisation d'un examen immunohistochimique (IHC) pour identifier la présence ou l'absence de p16^{INK4A} dans un échantillon de la tumeur.

10. Anticorps se liant spécifiquement à un polypeptide EGFr, pour l'utilisation selon la revendication 6 ou 7, dans lequel la tumeur est une tumeur localement avancée ou une tumeur métastatique récidivante, de préférence dans lequel la tumeur est sélectionnée dans le groupe comprenant une tumeur de l'oropharynx, une tumeur du larynx, une tumeur de la cavité buccale et une tumeur de l'hypopharynx.

11. Anticorps se liant spécifiquement à un polypeptide EGFr, pour l'utilisation selon la revendication 6 ou 7, dans lequel une chimiothérapie est utilisée en plus de l'anticorps qui se lie spécifiquement à un polypeptide EGFr.

12. Procédé de stratification d'une population de patients atteints d'une tumeur, comprenant :
(a) déterminer si la tumeur du patient est positive à HPV ou négative à HPV ; et
(b) sélectionner des patients dont les tumeurs sont négatives à HPV pour les traiter avec une thérapie comprenant un agent qui se lie spécifiquement à un polypeptide EGFr.

13. Procédé selon la revendication 12, dans lequel la détermination comprend le fait de déterminer la présence ou l'absence de p16^{INK4A} dans une tumeur prélevée sur le patient, dans lequel la présence de p16^{INK4A} indique que le patient est positif à HPV et l'absence de p16^{INK4A} indique que le patient est négatif à HPV.

14. Procédé selon la revendication 12, dans lequel l'agent qui se lie spécifiquement à un polypeptide EGFr est un anticorps du EGFr, de préférence dans lequel l'anticorps du EGFr est un panitumumab.

15. Procédé selon la revendication 12, dans lequel la détermination de la présence ou de l'absence de p16^{INK4A} dans une tumeur comprend la réalisation d'un examen immunohistochimique (IHC) pour identifier la présence ou l'absence de p16^{INK4A} dans un échantillon de la tumeur.

16. Procédé selon la revendication 12, dans lequel la tumeur est une tumeur localement avancée ou une tumeur métastatique récidivante, de préférence dans lequel la tumeur est sélectionnée dans le groupe comprenant une tumeur de l'oropharynx, une tumeur du larynx, une tumeur de la cavité buccale et une tumeur de l'hypopharynx.
